# EUROPÄISCHE PATENTSCHRIFT

(11) **EP 1 599 242 B1**
(45) Veröffentlichungstag und Bekanntmachung des Hinweises auf die Patenterteilung: **23.04.2008**
(21) Anmeldenummer: 04711566.2
(22) Anmeldetag: 17.02.2004
(51) Int. Cl.: A61M 5/30

(54) **VORRICHTUNG ZUM INJIZIEREN EINES STAUB- ODER PULVERFÖRMIGEN STOFFS IN EIN GEWEBE EINES KÖRPERS**
DEVICE FOR INJECTING A DUST-LIKE OR POWDERY SUBSTANCE INTO A BODY TISSUE
DISPOSITIF POUR INJECTER UNE SUBSTANCE PULVERULENTE OU POUDREUSE DANS LE TISSU D'UN CORPS

(30) Priorität: 17.02.2003 DE 10306716
(43) Veröffentlichungstag der Anmeldung: 30.11.2005
(73) Patentinhaber: Lell, Peter, Dr., 85368 Moosburg (DE)
(72) Erfinder: Lell, Peter, Dr., 85368 Moosburg (DE)
(74) Vertreter: Eder, Thomas
(86) Internationale Anmeldenummer: PCT/DE2004/000290
(87) Internationale Veröffentlichungsnummer: WO 2004/071558

(56) Entgegenhaltungen:
- WO-A-01/05451
- WO-A-01/97880
- WO-A-02/07803
- WO-A-96/25190
- US-A- 6 074 360
- US-A1- 2002 091 353

## Beschreibung

Die Erfindung betrifft eine Vorrichtung zum Injizieren eines staub- oder pulverförmigen Stoffs in ein Gewebe eines Körpers, insbesondere eines staub- oder pulverförmigen Medikaments oder nackter DNA.

In jüngerer Zeit wurden Vorrichtungen zum nadellosen Injizieren von vornehmlich flüssigen Stoffen entwickelt, welche den flüssigen Stoff unter hohem Druck in das betreffende Gewebe injizieren. Darüber hinaus wurden auch nadellose Injektionsvorrichtungen entwickelt, die es ermöglichen, staub- oder pulverförmige Stoffe in ein Gewebe zu injizieren. Eine derartige Vorrichtung ist unter der Bezeichnung PowderJect, hergestellt von der gleichnamigen englischen Firma, auf dem Markt, bei der der in einer Kammer gehaltene pulverförmige Stoff von Heliumgas unter Zerstörung der Wandungen der Kammer mitgerissen und mittels einer nachfolgenden Düse in Richtung auf das Gewebe beschleunigt wird. Mit einer derartigen Vorrichtung lassen sich Dosen von bis zu 2 oder 3 mg auf eine Zielfläche von bis zu 2 cm² beschleunigen. Abhängig vom Gasdruck, der Ausbildung der Düse, der Teilchengröße und deren Dichte bzw. Härte oder Festigkeit dringen die Teilchen dann in das Gewebe ein. Das Heliumgas ist unter hohem Druck in einem im Gehäuse der Vorrichtung vorgesehenen Behälter angeordnet. Mittels eines Auslösemechanismus wird der Behälter geöffnet und das Gas tritt unter hohem Druck aus, wodurch die Wandungen der Kammer, in welcher der Stoff gehalten ist zerstört werden.

Nachteilig bei dieser Anordnung ist die Verwendung des nur sehr schwierig über längere Zeit dicht einschließbaren Heliums sowie die relativ geringe Zuverlässigkeit bei der Öffnung des Heliumbehälters.

Des Weiteren wurden Vorrichtungen zur Injektion eines staub- oder pulverförmigen Stoffs entwickelt, bei der zur Beschleunigung der Partikel in Richtung auf das Gewebe ein Gasgenerator verwendet wird. Derartige Vorrichtungen sind beispielsweise in den internationalen Patentanmeldungen WO-A-01/41839, WO-A-01/47585 und WO-A-01/56637 beschrieben. Bei diesen bekannten Vorrichtungen wird ein pyrotechnischer Gasgenerator verwendet, welcher das Transportgas für den zu injizierenden staub- oder pulverförmigen Stoff erzeugt.

Bei der Vorrichtung gemäß WO-A-01/47585 ist der zu injizierende staub- oder pulverförmige Stoff in einer Kammer enthalten, welche auf einer Seite von einer Membran und auf der anderen Seite von der Wandung eines verschiebbaren Kolbens begrenzt ist. Der verschiebbare Kolben wird durch den Druck des vom Gasgenerator erzeugten Gases nach vorne bewegt, solange bis sich eine Öffnung des hohlzylindrischen Kolbens in den Bereich der Kammer verschoben hat, in welcher der zu injizierende Stoff enthalten ist. Der Gasdruck beaufschlagt zudem die Membran dieser Kammer und führt bei Überschreiten eines Schwelldrucks zu einer Zerstörung der Membran. Auf diese Weise wird der zu injizierende Stoff infolge des Gasdrucks durch die Öffnung des verschiebbaren Kolbens in Richtung auf die Austrittsöffnung der Vorrichtung transportiert.

Bei der Vorrichtung gemäß WO-A-01/41839 ist dem Gasgenerator und einer diesem nachgeschalteten Expansionskammer eine Kammer zur Aufnahme des zu injizierenden staub- oder pulverförmigen Stoffs nachgeordnet. Diese Kammer ist von zwei Membranen verschlossen, die den im Wesentlichen zylindrischen Raum für den Stoff zwischen der Expansionskammer und einem nachgeschalteten Düsenbereich begrenzen. Die beiden Membrane werden durch den Gasdruck zerstört und der zu injizierende Stoff durch das erzeugte Gas in Richtung auf eine Ausstoßöffnung der Vorrichtung mitgerissen. Um Partikel, die bei der Erzeugung des relativ heißen Gases entstehen, nicht ebenfalls auszustoßen, ist zwischen Gasgenerator und der Expansionskammer ein Filter vorgesehen.

Auch bei der nadellosen Injektionsvorrichtung nach der WO-A-01/56637 findet ein Gasgenerator Verwendung, welchem eine Expansionskammer und anschließend eine Kammer zur Aufnahme des zu injizierenden Materials nachgeschaltet ist. Die Kammer zur Aufnahme des zu injizierenden Stoffs ist durch eine erste Membran gebildet, welche an der der Expansionskammer abgewandten Seite eine sich in der Längsachse der Vorrichtung erstreckende geschlossene zylindrische Wandung aufweist. Diese geschlossene Wandung bildet die Kammer zur Aufnahme des Stoffs. Die offene Seite dieser Kammer ist durch eine zweite Membran begrenzt. Bei Erzeugung eines Gasdrucks birst die erste Membran und durchstößt mit der geschlossenen Wandung ebenfalls die zweite Membran, so dass der zu injizierende Stoff vom erzeugten Gas in Richtung auf die Ausstoßöffnung mitgerissen wird.

Derartige Vorrichtungen sind für das Injizieren relativ großer Mengen von staub- oder pulverförmigen Stoffen geeignet. Nachteilig ist dabei der Aufwand zur Erzeugung einer Kammer zur Aufnahme des zu injizierenden Stoffs, die möglichst definiert bei Erzeugung des Gasdrucks geöffnet werden muss.

Aus der US 2002/0091353 ist eine Vorrichtung gemäß dem Oberbegriff des Anspruchs 1 bekannt.

Dem gegenüber liegt der Erfindung die Aufgabe zu Grunde, eine Vorrichtung zum nadellosen Injizieren eines staub- oder pulverförmigen Stoffs in ein Gewebe eines Körpers zu schaffen, welche mit geringem Aufwand herstellbar ist und insbesondere das einfache Injizieren geringer Mengen eines staub- oder pulverförmigen Stoffs in das betreffende Gewebe ermöglicht.

Die Erfindung löst diese Aufgabe mit den Merkmalen des Patentanspruchs 1.

Die Erfindung geht von der Erkenntnis aus, dass geringe Mengen eines staub- oder pulverförmigen Stoffs auf einfache Weise an der Oberfläche einer Membran aufgebracht werden können. Die Partikelgröße des staub- oder pulverförmigen Stoffs liegt dabei vorzugsweise in einem Bereich < 10 µm. In diesem Bereich genügen allein die Adhäsionskräfte, um geringe definierte Mengen eines derartigen Stoffs auf der Membran, die beispielsweise aus einem Kunststoff oder Metall bestehen kann, zu halten.

Insbesondere für größere Partikelgrößen kann es jedoch notwendig werden, einen Haftvermittler auf der Oberfläche der Membran vorzusehen. Hierbei kann es sich um einen Klebstoff, ein Gel oder ein Öl beispielsweise Silikonöl, handeln.

In der Vorrichtung ist an der rückwärtigen Seite der Membran ebenfalls ein Gas erzeugendes Material vorgesehen, mit dessen Hilfe ein definierter Druckstoß oder definierter Impuls mittelbar oder unmittelbar auf die Membran übertragbar ist. Der auf die Membran übertragene Druckstoß ist dabei durch die Beschaffenheit des Gas erzeugenden Materials und die Beschaffenheit und Ausbildung der Membran sowie weitere Dimensionierungsparameter des Gehäuses (Größe einer Expansionskammer für das erzeugte Gas, Vorsehen eines Co-Volumens und dergleichen) so bestimmt, dass ein ausreichender Impuls auf den auf der Oberfläche der Membran vorgesehenen staub- oder pulverförmigen Stoff übertragen wird. Dieser auf den Stoff übertragene Impuls führt dazu, dass sich die Stoffpartikel von der Membran ablösen und so hoch beschleunigt werden, dass die Stoffpartikel eine ausreichende kinetische Energie aufweisen, um mindestens eine vorbestimmte Tiefe in das Gewebe eindringen zu können.

Das Gas erzeugende Material kann dabei insbesondere ein pyrotechnisches Material sein. Als Gas erzeugendes Material wird dabei auch ein explosionsfähiges Gasgemisch verstanden, das bei der Umsetzung Energie und eine andere Gaszusammensetzung bzw. zuvor nicht vorhandene Gasarten erzeugt. Das Gas erzeugende Material kann auch eine Flüssigkeit sein, die bei Vorhandensein eines Katalysator vergast.

Nach einer Ausführungsform der Erfindung kann das Gas erzeugende Material in einer Kammer des Gehäuses angeordnet sein, wobei nach dem Aktivieren des Gas erzeugenden Materials ein Druckstoß erzeugt wird. Dieser Druckstoß kann unmittelbar auf die Membran übertragen werden und bei Auftreffen auf die Membran einen ausreichenden Impuls auf diese übertragen. Unter Impuls wird dabei im Sinne dieser Beschreibung nicht nur die streng physikalische Definition (Masse x Geschwindigkeit eines Körpers), sondern ganz allgemein eine stoßartige Kraft- oder Druckeinwirkung verstanden.

Nach einer anderen Ausführungsform der Erfindung kann mittels des erzeugten Druckstoßes oder mittels eines ansteigenden Gasdrucks ein im Gehäuse geführtes Kolbenelement beaufschlagt werden, welches hierdurch so hoch beschleunigt wird, dass es bei einem Auftreffen auf die Membran den erforderlichen Impuls auf diese überträgt.

Das Kolbenelement kann dabei so ausgebildet sein, dass es auch während seiner Verschiebebewegung den Raum, dem es mit seiner durch den Gasdruck beaufschlagten Seite zugewandt ist, abdichtet, wobei das Kolbenelement hierzu vorzugsweise im Bereich dieser Seite selbstlidernd und damit abdichtend ausgebildet ist. Unter Selbstliderung wird hier eine Ausbildung des Kolbenelements verstanden, bei dem ein einstückig mit dem Kolbenelement ausgebildeter Randbereich eine dünne Wandung aufweist, welche vom Gasdruck beaufschlagt und in abdichtender Weise radial nach außen an die Innenwandung des Bereichs gedrückt wird, in dem das Kolbenelement verschiebbar geführt ist.

Das Kolbenelement kann in einer Ausgangsstellung so im Gehäuse gehalten oder angeordnet sein, dass es erst bei Beaufschlagung mit einem, einen Schwellenwert übersteigenden Druck seine Haltekraft überwindet und schlagartig beschleunigt wird. Selbstverständlich kann das Kolbenelement auch so ausgebildet sein, dass es mit einem Haltebereich fest im Gehäuse gehalten ist und einen damit einstückig verbundenen Kolbenbereich aufweist, welcher bei Beaufschlagung mit einem einen Schwellwert überschreitenden Druck ausbricht.

Das Kolbenelement kann auch so ausgebildet sein, dass es im Raum zwischen ihm, d. h. seiner Vorderseite bezogen auf die Bewegungsrichtung des Kolbenelements, und der Membran einen Druckanstieg oder einen Druckstoß erzeugt. Dieser Druckstoß oder Druckanstoß kann dann auf die Membran wirken. Der Kolben muss sich dabei nicht notwendigerweise so weit nach vorne bewegen, dass er ebenfalls auf die Membran auftrifft.

Die Membran ist ortsfest im Gehäuse gehalten und so ausgebildet, dass sie infolge des auf sie wirkenden Druckanstiegs oder Druckstoßes oder durch das auf sie auftreffende Kolbenelement nicht zerstört wird. Die Membran kann hierzu beispielsweise aus einer dünnen Metallplatte bestehen. Zur Ablösung und Beschleunigung des auf der der Austrittsöffnung zugewandten ebenen Oberfläche der Membran gehaltenen Partikels des zu injizierenden Stoffs bestehen unterschiedliche Möglichkeiten bzw. Mechanismen, die nachstehend erläutert werden und die auch in Kombination miteinander auftreten können.

Zum einen besteht die Möglichkeit, die Membran so auszubilden, dass die Schallgeschwindigkeit innerhalb des Membranmaterials in der Ausstoßrichtung kleiner ist als die Geschwindigkeit eines erzeugten und auf die Membran auftreffenden Druckstoßes. Der Druckstoß geht in diesem Fall durch das Material der Membran hindurch und erzeugt einen Impuls bzw. eine Beschleunigung der Partikel des zu injizierenden Materials, die sich dabei von der Oberfläche der Membran lösen. Die Membran kann dabei vollkommen steif und mit einer relativ großen Dicke ausgebildet sein. Eine derartige Membran kann beispielsweise aus einer Stahlplatte mit einer Dicke von einem 1mm und mehr bestehen. Ein derartiger Druckstoß kann durch ein sehr lebhaft abrennendes pyrotechnisches Material oder einen detonativen Stoff erzeugt werden. Von einem detonativen Stoff wird gesprochen, wenn bei dessen Aktivierung ein Druckstoß mit einer Geschwindigkeit von mindestens 2000 m/s erzeugt wird.

Der auf die Partikel zu übertragende Impuls muss dabei mindestens so groß sein, dass die Partikel eine Auftreffgeschwindigkeit auf das Gewebe von ca. 800 bis 1000 m/s aufweisen.

Ein weiterer Mechanismus zur Ablösung und Beschleunigung der Partikel des zu injizierenden Stoffs von der Membranoberfläche ohne eine Zerstörung der Membran besteht darin, dass die Membran durch den auf sie wirkenden Druckanstieg oder Druckstoß so deformiert wird, dass bei einer Verzögerung der Deformationsbewegung der Membran so hohe Beschleunigungskräfte auf die Partikel des zu injizierenden Stoffs wirken, dass die Partikel von der Membranoberfläche abgelöst werden und die Partikel noch mit einer ausreichend hohen Geschwindigkeit ausgestoßen werden.

Die Membran kann nach einer Ausführungsform der Erfindung dabei so ausgebildet sein, dass der Bereich der Membran, in dem der zu injizierende staub- oder pulverförmige Stoff aufgebracht ist, so dick ausgebildet ist, dass im Wesentlichen keine Deformation dieses Bereichs bei einer Übertragung des vom Gas erzeugten Impulses auf die Membran erfolgt. Beispielsweise kann der Randbereich der Membran, in dem diese im Gehäuse gehalten ist, relativ dünn ausgebildet sein, so dass die Membran in diesem dünnwandigeren Bereich deformiert wird. Dies hat den Vorteil, dass der gesamte Bereich, in dem der zu injizierende Stoff aufgebracht ist, gleichmäßig beschleunigt und dann wieder verzögert wird. Die Partikel werden daher im praktisch gesamten Bereich gleichmäßig abgelöst und weisen in etwa die selbe Ausstoßgeschwindigkeit nach dem Ablösen von der Membran auf.

Vorraussetzung für eine Nutzung dieses Ablösungsmechanismus ist damit eine ausreichend deformierbare Membran. Die Deformation kann nur in bestimmten Bereichen oder über die gesamte Membran auftreten. Dabei ist sowohl eine elastische als auch plastische Deformation möglich.

Wird die Membran unmittelbar von dem erzeugten Gas beaufschlagt, so wird für das Gas erzeugende Material vorzugsweise Tetrazen oder eine aktivierbare Stoffmischung verwendet, welche im Wesentlichen nur Stickstoff erzeugt. Selbstverständlich ist auch der Einsatz einer Filtereinrichtung oder Rückhalteeinrichtung für bei der Gaserzeugung entstehende Partikel oder Heißgaspartikel möglich.

Bei Verwendung eines Kolbens kann die Vorrichtung so ausgebildet sein, dass die Membran entweder durch den Kolben selbst oder durch einen Druckanstieg erzeugt wird, welcher infolge des Verschiebens des Kolbens in Ausstoßrichtung vor dem Kolben entsteht.

Die Vorrichtung weist in Ausstoßrichtung nach der Membran vorzugsweise einen Düsenbereich auf, der zur Beschleunigung des die Partikel tragenden Gasstroms dient.

Nach einer Ausführungsform der Erfindung kann in Ausstoßrichtung vor dem Düsenbereich ein Auffangbereich für das Kolbenelement vorgesehen sein, welcher vorzugsweise in Ausstoßrichtung verjüngend ausgebildet ist. Das Kolbenelement und der Auffangbereich sind dabei vorzugsweise so aufeinander abgestimmt, dass das Kolbenelement nach dem Auffangvorgang abdichtend im Auffangbereich gehalten ist. Auf diese Weise wird verhindert, dass das erzeugte Gas mit den auszustoßenden Partikeln in Berührung kommt. Diese werden ausschließlich durch das Gasvolumen (beispielsweise Luft-, Helium-, oder Wasserstoffvolumen) getragen, welches sich im Ausgangszustand vor dem Kolbenelement befindet.

Das Kolbenelement kann aus einem deformierbaren Material bestehen, wobei der Auffangbereich und das Kolbenelement so aufeinander abgestimmt sind, dass sich das Kolbenelement während des Verzögerns im Auffangbereich unter Reduzieren des Durchmessers deformiert. Hierdurch wird zusätzlich zur abdichtenden Wirkung erreicht, dass sich das Kolbenelement verjüngt und demzufolge die vordere Stirnseite des Kolbens während des Verzögerungsvorgangs gegenüber dem Massenschwerpunkt des Kolbenelements zunächst noch beschleunigt wird. Hierdurch wird eine zusätzliche Beschleunigung der mittels des Kolben ausgetriebenen Gase bzw. erreicht. Der Auffangbereich ist dabei hinsichtlich seiner Verjüngung so ausgebildet, dass die Stirnseite des Kolbens in dem Zeitpunkt auf die Membran auftrifft, in dem die Stirnseite gerade die maximale Geschwindigkeit aufweist.

Nach einer Ausführungsform der Erfindung kann das Gas erzeugende Material in Form eines separat ausgebildeten Detonators oder eines separat ausgebildeten Anzündstücks vorgesehen sein. Selbstverständlich kann zusätzlich zu einem Detonator oder einem Anzündstück ein weiteres pyrotechnisches Material oder Energie lieferndes Material vorgesehen sein, welches durch die vom Detonator bzw. vom Anzündstück erzeugten Heißgase angezündet wird.

Anstelle eines Detonators oder Anzündstücks, die vornehmlich (jedoch nicht ausschließlich) elektrisch aktivierbar sind, kann auch ein Zündstift verwendet werden, um ein zusätzliches Gas erzeugendes Material zu aktivieren. Eine derartige Zündeinheit umfasst einen Trägerstift, auf welchem (vorzugsweise auf dessen zylindrischer Umfangsfläche) ein stoßempfindliches pyrotechnisches Material in Form einer Beschichtung aufgebracht ist. Der Trägerstift selbst wird in ein dünnwandiges, einseitig verschlossenes Röhrchen eingeführt, um das pyrotechnische Material auf dem Trägerstift gegen Umwelteinflüsse zu schützen. Gleichzeitig dichtet das Röhrchen die Brennkammer gegen die Umgebung ab. Dieses Material kann mittels eines kurzen und schnellen Schlags auf das Röhrchen, das sich hierbei leicht verformt, aktiviert werden.

Die Vorrichtung kann auch einen separat ausgebildeten und austauschbar im Gehäuse gehaltenen Gasgenerator umfassen, welcher ein aktivierbares Material und eine Zündeinrichtung, beispielsweise einen Detonator, ein Anzündstück oder eine Zündeinheit umfasst.

In einer weiteren Ausführungsform der Erfindung kann das Gas erzeugende Material auch direkt auf der der Ausstoßöffnung abgewandten Oberfläche der Membran angeordnet sein. Dieses Material ist vorzugsweise so gewählt, dass es mittels eines kurzen thermischen oder mechanischen Impulses aktivierbar ist. Die bei einer Aktivierung erzeugten Rückstoßkräfte wirken impulsartig auf die Membran und führen zu einem der vorstehend erläuterten Mechanismen zur Ablösung der Partikel des zu injizierenden Stoffs von der Membranoberfläche, ohne dass eine Zerstörung der Membran erforderlich wäre.

Weitere Ausführungsformen der Erfindung ergeben sich aus den Unteransprüchen.

Die Erfindung wird nachfolgend an Hand in der Zeichnung dargestellter Ausführungsbeispiele der Fig. 1-5 näher erläutert. Die Beispiele der Fig. 6-9 fallen nicht unter den Wortlaut des Anspruchs 1. In der Zeichnung zeigen
- Fig. 1: einen Längsschnitt durch eine erste Ausführungsform einer Vorrichtung zur nadellosen Injektion eines staub- oder pulverförmigen Stoffs mit einer ebenen Membran konstanter Dicke;
- Fig. 2: eine Ausführungsform ähnlich Fig. 1, jedoch mit verdickter Membran im Bereich des Belags mit dem zu injizierenden Stoff;
- Fig. 3: eine schematische Darstellung einer mittels einer Treiberplatte beaufschlagten Membran;
- Fig. 4: einen Längsschnitt durch eine weitere Ausführungsform einer wiederverwendbaren Vorrichtung mit separatem Gasgenerator und einem verschiebbaren Kolben zur Impulserzeugung;
- Fig. 5: eine Ausführungsform ähnlich Fig. 4 mit einem verschiebbaren Kolben und einem weiteren ausbrechbaren Kolben zur Erzeugung eines steilen Impulses;
- Fig. 6: ein Beispiel ähnlich Fig. 1, jedoch mit zerstörbarer Membran und nachgeordnetem Düsenbereich;
- Fig. 7: ein Beispiel ähnlich Fig. 4, jedoch mit zerstörbarerer Membran und nachgeordnetem Düsenbereich;
- Fig. 8: ein Bespiel ähnlich Fig. 7, wobei der Kolben vor Auftreffen auf die Membran einen Druckanstieg erzeugt und
- Fig. 9: ein Beispiel ähnlich Fig. 3, jedoch mit einem Gas erzeugenden Material an der rückwärtigen Oberfläche der Membran und mechanischer Aktivierungseinrichtung.

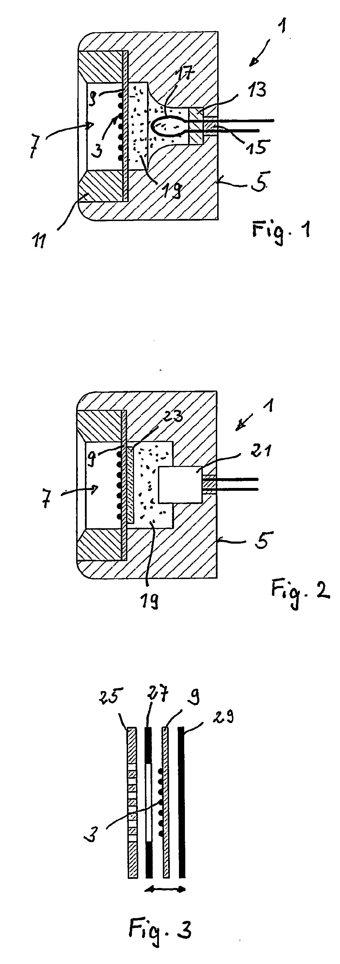

Die in Fig. 1 dargestellte Vorrichtung 1 zum nadellosen Injizieren eines staub- oder pulverförmigen Stoffs 3 in ein nicht näher dargestelltes Gewebe eines Körpers, beispielsweise eines menschlichen Patienten, umfasst ein Gehäuse 5, welches im Wesentlichen zylindrisch ausgebildet ist und einen zentralen Durchbruch aufweist. Im vorderen Bereich des Gehäuses 5 bildet der Durchbruch eine Ausstoßöffnung 7 für den zu injizierenden Stoff 3, welcher auf der der Ausstoßöffnung 7 zugewandten Oberfläche einer Membran 9 aufgebracht ist. Die Membran 9 kann beispielsweise aus Kunststoff, einem Faserverbundwerkstoff (insbesondere Kohle- oder Bohrfaser) oder einem Metall, z.B. Stahl bestehen und, wie in Fig. 1 dargestellt, als ebenes kreisrundes Plättchen ausgebildet sein, welches mittels eines in das Gehäuse 5 einschraubbaren Halterings 11 fixiert ist.

Der zentrale Durchbruch des Gehäuses 5 ist am rückwärtigen Ende mittels einer Dichtplatte 13 und einem Stopfen 15 dicht verschlossen, wobei durch den Stopfen 15 die Anschlüsse eines Glühdrahts oder einer Glühwendel 17 geführt sind. Der Glühdraht 17 erstreckt sich in den rückwärtigen Bereich des Raums des zentralen Durchbruchs und dient als Anzündeinrichtung für ein in diesem Bereich eingebrachtes Gas erzeugendes Material 19. Das Gas erzeugende Material 19 kann ein pyrotechnisches Material sein. Wird durch die Anschlüsse des Glühdrahts 17 ein ausreichend großer elektrischer Strom geleitet, beispielsweise mittels einer nicht näher dargestellten elektrischen Aktivierungseinrichtung, so wird durch die vom Glühdraht abgegebene thermische Energie das Gas erzeugende Material 19 aktiviert. Das Material 19, welches beispielsweise als pyrotechnisches Material ausgebildet sein kann, setzt nach dem Aktivieren um und erzeugt im Raum zwischen der Rückseite der Membran 9 und der Vorderseite der Dichtplatte 13 einen schnell ansteigenden Druckverlauf oder einen Druckstoß. Die Geschwindigkeit des Druckanstiegs bzw. die Ausbreitungsgeschwindigkeit des Druckstoßes kann durch eine entsprechende Wahl des Gas erzeugenden Materials 19 hinsichtlich seiner Umsetzungseigenschaften und seiner Menge sowie durch die Formgebung und Dimensionierung des Raums, in welchem das Material vorgesehen ist bzw. in welchem der Gasdruck bzw. der Druckstoß erzeugt wird, festgelegt werden.

Bei dem in Fig. 1 dargestellten Ausführungsbeispiel wird man als Gas erzeugendes Material beispielsweise ein sehr lebhaft umsetzendes deflagrierendes pyrotechnisches Material wählen, welches einen ausreichend steilen Druckanstieg erzeugt. Durch den Druckanstieg werden entsprechend hohe Kräfte auf die rückseitige Oberfläche der Membran ausgeübt. Diese wird sich dementsprechend nach vorne wölben, bis eine maximale Deformation der Membran 9 erreicht ist. Bei einem entsprechend schnellen Druckanstieg, welcher impulsartig auf die Membran 9 wirkt, wird diese in entsprechende schnelle Bewegungen versetzt werden. Die Amplitude der Bewegungen ist abhängig vom erzeugten Druckverlauf in der Kammer, welche mittels der Membran verschlossen ist sowie durch das Material, die Dicke und die Formgebung der Membran.

Unabhängig davon, ob letztendlich eine plastische oder elastische Deformation vorliegt, werden die Bereiche der Membran, in welchen der zu injizierende staub- oder pulverförmige Stoff 3 aufgebracht ist, zunächst in Richtung auf die Ausstoßöffnung 7 beschleunigt und dann verzögert. Durch den impulsartig erzeugten Druckanstieg treten derart hohe Beschleunigungs- und Verzögerungswerte auf, dass sich die Partikel des Stoffs 3 von der Oberfläche der Membran 9 ablösen und mit einer ausreichend hohen Geschwindigkeit durch die Ausstoßöffnung 7 in Richtung auf das Gewebe, in welches injiziert werden soll, ausgestoßen werden.

Wie bereits erwähnt, ist die Membran bei der in Fig. 1 dargestellten Ausführungsform so dimensioniert, dass infolge des Druckanstiegs oder Druckstoßes keine Zerstörung der Membran erfolgt, sondern eine entsprechende Beschleunigung und Verzögerung derjenigen Membranbereiche, in welchen der zu injizierende Stoff 3 auf der Oberfläche der Membran aufgebracht ist. Die Membran und das Gas erzeugende Material sind dabei so aufeinander abgestimmt, dass sich die Partikel des zu injizierenden Stoffs 3 von der Membranoberfläche ablösen und mit einer Geschwindigkeit ausgestoßen werden, die ca. 800 m/s oder mehr beträgt. Diese Geschwindigkeit ist erforderlich, um eine ausreichende Eindringtiefe der Partikel in das jeweilige Gewebe zu erreichen.

Es sei darauf hingewiesen, dass der Raum, welcher mit der Membran 9 verschlossen ist, selbstverständlich nicht vollständig mit einem Gas erzeugenden Material 19 ausgefüllt werden muss. Vielmehr kann die Menge des Materials 19 an die jeweiligen Erfordernisse angepasst werden. Auch sind selbstverständlich beliebige andere Aktivierungseinrichtungen zur Aktivierung des Gas erzeugenden Materials möglich. Beispielsweise kann eine Zündeinheit verwendet werden, welcher in den Raum mit dem Gas erzeugenden Material 19 hereinragt und der beispielsweise mittels der Dichtplatte 13 und dem Stopfen 15 gehalten ist. Mittels einer mechanischen Vorrichtung kann dann ein kurzer Schlag mit ausreichender Energie auf das Röhrchen der Zündeinheit ausgeübt werden. Das auf der vorzugsweise zylindrischen Außenwandung des Trägerstifts aufgebrachte, erschütterungsempfindliche pyrotechnische Material wird durch den Schlag aktiviert und setzt um. Hierdurch wird das Gas erzeugende Material 19 aktiviert bzw. angezündet. Antelle mittels eines Schlags kann das Material auch mittels Reibung aktiviert werden.

Die in Fig. 2 dargestellte weitere Ausführungsform einer Vorrichtung 1 zum nadellosen Injizieren eines staub- oder pulverförmigen Stoffs unterscheidet sich von der Ausführungsform in Fig. 1 im Wesentlichen dadurch, dass im rückwärtigen Bereich des Gehäuses 5 ein Anzündstück oder ein Detonator 21 abgedichtet gehalten ist. Elektrische Anschlussleitungen des Anzündstücks oder Detonators 21 sind wieder an der Rückseite des Gehäuses 5 herausgeführt.

Ist ein Anzündstück 21 vorgesehen, so kann der Raum an der Rückseite der Membran 9 wiederum zumindest teilweise mit einem Gas erzeugenden Material, beispielsweise einem pyrotechnischen Material 19 gefüllt. Bei einer elektrischen Aktivierung des Anzündstücks wird das darin vorgesehene pyrotechnische Material aktiviert und durch dessen thermische Energie das Material 19 aktiviert.

Die Membran 9 der Ausführungsform in Fig. 2 ist in dem Bereich, in dem an der der Austrittsöffnung 7 zugewandten Oberfläche der zu injizierende Stoff 3 aufgebracht ist, verdickt ausgebildet. Hierdurch wird eine größere Steifheit der Membran in diesem Bereich erreicht. Im Übrigen ist die Funktionsweise dieser Ausführungsform mit der Funktionsweise, wie sie vorstehend in Verbindung mit der Ausführungsform nach Fig. 1 erläutert wurde, identisch. Die Deformation der Membran erfolgt jedoch nunmehr im Wesentlichen nur in den Bereichen zwischen der Verdickung und der Einspannung der Membran im Gehäuse 5. Auf diese Weise wird erreicht, dass der verdickte Bereich der Membran im Wesentlichen konstant beschleunigt und verzögert wird und somit alle Partikel, die auf der Oberfläche diese Bereichs angeordnet sind, mit im Wesentlichen der selben Geschwindigkeit ausgestoßen werden. Dagegen werden bei der Ausführungsform nach Fig. 1 die Partikel des Stoffs 3, die in der Achse der Vorrichtung angeordnet sind, mit einer höheren Geschwindigkeit ausgestoßen, da sich dieser Bereich infolge des Gasdrucks oder Druckstoßes stärker wölben wird als die Randbereiche und demzufolge die Beschleunigungs- und Verzögerungskräfte in diesem Bereich größer sind.

Wie in Fig. 2 dargestellt, kann die Verdickung der Membran in denjenigen Bereichen, auf deren Oberfläche der Stoff 3 aufgebracht ist, durch eine Verstärkungsplatte 23 erreicht werden. Selbstverständlich kann die Membran jedoch auch einstückig mit einer entsprechenden Verdickung ausgebildet sein. Nach einer weiteren, nicht näher dargestellten Ausführungsform kann auch eine Membran mit konstanter, relativ hoher Dicke verwendet werden, welche eine ringförmigen Ausnehmung an einer oder beiden Oberflächen aufweist, wodurch im Bereich der Ausnehmung(en) eine Verjüngung der Materialdicke erreicht wird. In diesen Bereichen wird dann wiederum die elastische oder plastische Deformation vornehmlich auftreten.

Bei der Ausführungsform nach Fig. 2 kann auch ein Detonator 21 eingesetzt werden, welcher ein detonativ umsetzendes pyrotechnisches Material enthält. Bei einem Aktivieren eines derartigen Detonators wird ein Stoßwelle erzeugt, der sich mit einer entsprechend hohen Ausbreitungsgeschwindigkeit (beispielsweise größer als 2000 m/s) in Richtung auf die Rückseite der Membran 9 zubewegt. In diesem Fall muss kein zusätzliches Gas erzeugendes Material 19 im Raum zwischen der Membran 9 und dem Detonator 21 enthalten sein. Diese Stoßwelle läuft durch das Material der Membran 9 hindurch und wird auf die Partikel des Stoffs 3 übertragen. Es ist dabei nicht erforderlich, dass die Membran (makroskopisch) deformiert wird. Die Membran kann in diesem Fall relativ dick und steif ausgebildet sein. Denn wenn die Schallgeschwindigkeit in der Membran in Ausstoßrichtung kleiner ist als die Geschwindigkeit des vom Detonator 21 erzeugten Druckstoßes, geht der Druckstoß mit nur geringer Dämpfung durch die Membran hindurch. Damit kann auch auf diese Weise ohne eine makroskopische Deformation der Membran ein Ablösen der Partikel des zu injizierenden Stoffs 3 von der Oberfläche der Membran 9 und ein Ausstoßen der Partikel mit einer ausreichend hohen Geschwindigkeit erreicht werden.

Selbstverständlich können diese beiden Mechanismen zum Ablösen und Beschleunigen der Partikel des zu injizierenden Stoffs 3 auch kombiniert auftreten.

An dieser Stelle sei darauf hingewiesen, dass das Aufhängen des zu injizierenden Stoffs 3 auf die Oberfläche der Membran 9 einfach dadurch erfolgen kann, dass der zu injizierende Stoff mit der Membranoberfläche in Kontakt gebracht wird. Insbesondere bei Partikelgrößen < 10 µm sind die Adhäsionskräfte zwischen den Partikeln und der Membranoberfläche ausreichend groß, um die Partikel an der Oberfläche zu fixieren.

Zusätzlich oder auch für größere Partikel kann auf der Membranoberfläche ein Haftvermittler aufgebracht sein, beispielsweise in Form eines Klebstoffs, Gels, oder eines Silikonöls. Die Vorrichtungen nach den Figuren 1 und 2 können beispielsweise als nur einmal verwendbare Vorrichtungen ausgebildet sein. Die Ausstoßöffnung der Vorrichtung kann hierbei beispielsweise mittels einer Schutzfolie verschlossen sein, die erst kurz vor dem Gebrauch abgenommen wird. Die Gehäuse 5 dieser Vorrichtungen können in das Gehäuse einer Haltevorrichtung eingesetzt werden, in welcher auch ein Teil der Aktivierungseinrichtung enthalten ist, beispielsweise eine elektrische Ansteuerung für elektrische Aktivierungseinrichtungen im Gehäuse 5.

Fig. 3 zeigt schematisch eine weitere, nicht im Rahmen der Erfindung liegende Möglichkeit zur Erzeugung eines ausreichend hohen Impulses auf eine Membran 9, um den auf deren Oberfläche aufgebrachten zu injizierenden Stoff 3 abzulösen und die betreffenden Partikel mit ausreichender Geschwindigkeit auszustoßen. Hierbei ist die Membran 9 in Pfeilrichtung, d. h. axial bzw. in Ausstoßrichtung verschiebbar in einem Gehäuse angeordnet bzw. geführt. Vor der Membran ist eine Anschlagplatte 25 vorgesehen, welche fest im Gehäuse gehalten ist. Zwischen Anschlagplatte 25 und Membran 9 ist ein kompressibles Element 27 vorgesehen, welches beispielsweise aus einem weichen Kunststoff bestehen kann. An der rückwärtigen Seite der Membran ist eine Prallplatte 29 angeordnet, auf welche die Druckkräfte wirken, die von dem Gas erzeugenden Material erzeugt werden. Die Elemente 25, 27, 9 und 29 können unmittelbar benachbart im Gehäuse gehalten sein. Bei einem Ausüben entsprechend hoher impulsartiger Druckkräfte auf die Prallplatte 29, welche ebenfalls zusammen mit der Membran 9 verschiebbar im Gehäuse gehalten ist, werden die Prallplatte 29 und die Membran 9 kurz in Ausstoßrichtung beschleunigt, wobei das kompressible Element 27 in seiner Dicke komprimiert wird. Diese Kompression kann elastisch oder plastisch folgen. In jedem Fall wird hierdurch die Membran 9 in Ausstoßrichtung beschleunigt und verzögert, wodurch wiederum ausreichend hohe Beharrungskräfte auf die Partikel des Stoffs 3 entstehen, die zu deren Ablösen führen.

Wie in Fig. 3 dargestellt, kann die Anschlagplatte 25 anstelle eines ausreichend großen zentralen Durchbruchs (wie auch das ringartige kompressible Element 27) eine Vielzahl kleiner Durchbrüche aufweisen, wodurch ein entsprechendes Injektionsmuster erzeugbar ist. Selbstverständlich kann die Prallplatte 29 auch als Kolben ausgebildet sein, welcher nicht unmittelbar an der Rückseite der Membran 9 anliegt, sondern zunächst mittels des erzeugten Gasdrucks beschleunigt wird und anschließend auf die Membran 9 auftrifft. In gleicher Weise kann auch das kompressible Element 27 entfallen oder zwischen dem Element 27 und der Membran 9 in der Ausgangslage ein vorbestimmter Abstand bestehen. Auf diese Weise wird zunächst die Membran verschoben, bevor sie auf den Anschlag auftrifft. Auch hierdurch lässt sich die vorstehend erläuterte Funktion erreichen. In der Regel wird entweder ein Druckstoß, eine Stoßwelle oder eine schnelle Masse auf die Prallplatte 29 auftreffen.

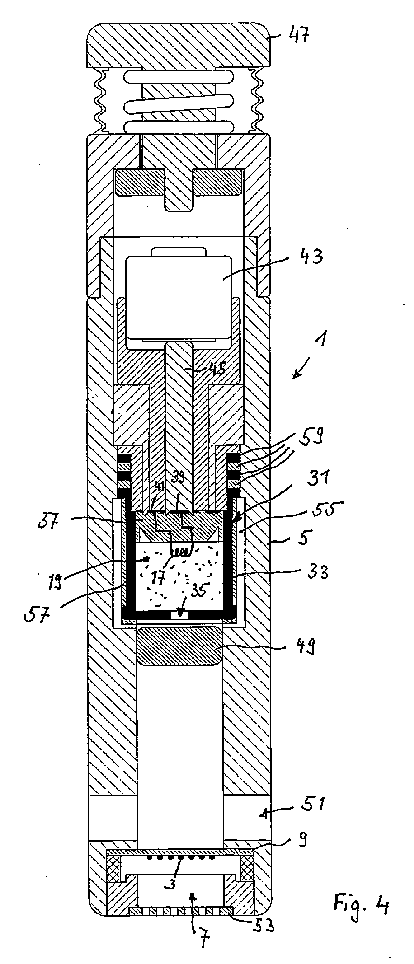

Die in Fig. 4 dargestellte Vorrichtung 1 zum nadellosen Injizieren eines staub- oder pulverförmigen Stoffs ist als mehrfach verwendbare Vorrichtung ausgebildet. In einem Gehäuse 5 ist ein Gasgenerator 31 vorgesehen, welcher austauschbar im Gehäuse gehalten ist. Der Gasgenerator 31 umfasst seinerseits ein Gehäuse 33, welches an seiner Vorderseite eine Austrittsöffnung 35 für das erzeugte Gas aufweist. An seiner Rückseite ist das Gehäuse 33 mittels einer abdichtenden Scheibe 37 verschlossen. An ihrer rückseitigen Oberfläche trägt die Scheibe 37 einen zentralen kreisförmigen elektrischen Kontakt 39 und einen ringförmigen Kontakt 41. Die Kontakte 39 bzw. 41 sind mit einem Glühdraht 17 verbunden, welcher sich in den Innenraum des Gehäuses 33 erstreckt. Der Innenraum des Gehäuses 33 ist wiederum zumindest teilweise mit einem Gas erzeugenden Material 19 gefüllt, welches mit dem Glühdraht 17 aktivierbar ist. Im rückwärtigen Ende der Vorrichtung 1 ist eine Batterie 43 gehalten, deren einer Kontakt dauernd über einen zentralen Stift 45 mit dem ringförmigen Kontakt 39 des Gasgenerators 31 in Verbindung steht. Der andere Kontakt der Batterie kann mittels einer Drucktaste 47 über elektrisch leitende Teile des Gehäuses 5 mit dem ringförmigen Kontakt 41 des Gasgenerators 31 in Verbindung gebracht werden.

Wird die Vorrichtung 1 auf das zu injizierende Gewebe aufgesetzt und anschließend die Drucktaste 47 betätigt, so wird der Gas erzeugende Stoff 19 mittels des Glühdrahts 17 angezündet. Das erzeugte Gas tritt über die Austrittsöffnung 35 aus und beaufschlagt einen vorzugsweise abdichtend im vorderen Bereich des Gehäuses 5 geführten Kolben 49. Dieser wird durch den Gasdruck beschleunigt und in Richtung auf die im vorderen Bereich des Gehäuses 5 gehaltene Membran 9 beschleunigt. Dabei sind im Gehäuse 5 in der Nachbarschaft der Rückseite der Membran 9 Ausströmöffnungen 51 vorgesehen, um die Beschleunigung des Kolbens 49 nicht infolge eines vor dem Kolben zu komprimierenden Gasvolumens zu behindern und um damit den Druckanstieg beim Auftreffen auf die Membran 9 steiler zu machen. Der Kolben 49 trifft mit einer definierten Geschwindigkeit auf die Membran 9 auf und überträgt damit eine impulsartige Kraft auf die Membran 9. Diese wird, wie vorstehend erläutert, deformiert, wodurch sich die Partikel des Stoffs 3 ablösen und durch die Ausstoßöffnung 7 austreten. Bei der in Fig. 4 dargestellten Ausführungsform ist im Bereich der Ausstoßöffnung, wie bereits im Zusammenhang mit Fig. 3 erläutert, eine plattenförmig Blende 53 vorgesehen, welche ein bestimmtes Muster von kleineren Durchbrüchen aufweist.

Die Position und Dimensionierung der Ausströmöffnungen 51 kann in Bezug auf die Dicke des Kolbens 49 so gewählt sein, dass die Ausströmöffnungen 51 vom Kolben verschlossen sind, nachdem dieser eine Endposition in unmittelbarer Nachbarschaft der Membran 9 erreicht hat. Auf diese Weise wird vermieden, dass das erzeugte Gas in die Umgebung austritt und sich störend oder gefährden auswirkt.

Gegebenenfalls kann die Lage der Ausströmöffnungen 51 auch so gewählt werden, dass ein vorbestimmter axialer Abstand zwischen den Ausströmöffnungen 51 und der Membran vorliegt. In diesem Fall erfolgt ein Komprimieren des Gasvolumens zwischen der Rückwandung der Membran und der Vorderseite des Kolbens, sobald der Kolben die Position der Ausströmöffnungen 51 (in Richtung der Ausströmöffnung) passiert hat. Hierdurch erfolgt bereits ein gewisses Druckbeaufschlagen bzw. Vorspannen der Membran 9, bevor der Kolben 49 auf diese auftrifft.

Gegebenenfalls kann auch vollständig auf Ausströmöffnungen 51 verzichtet werden. Im Extremfall erzeugt der Kolben 49 dann nur noch einen Druckstoß bzw. einen impulsartigen Druck, ohne selbst auf die Membran aufzutreffen.

Wie in Fig. 4 dargestellt, kann der Gasgenerator 31 so im Gehäuse 5 gehalten sein, dass dieser von einem Ringraum 55 umgeben ist. Dieser Ringraum kann als Co-Volumen dienen, in welches die erzeugten Gase ebenfalls eindringen. Durch die Wahl der Größe des Co-Volumens kann der Verlauf des Druckanstiegs und damit die Beschleunigung des Kolbens 49 gesteuert werden.

Selbstverständlich kann auch die Austrittsöffnung 35 des Gasgenerators 31 mittels einer zerstörbaren Membran verschlossen sein. Auf diese Weise lässt sich ein sehr steiler und schneller Druckanstieg erzeugen, abhängig von der Berstkraft der Membran.

Die Größe des Co-Volumens kann bei der Ausführungsform nach Fig. 4 dadurch verändert werden, dass unterschiedliche Gasgeneratoren mit einem unterschiedlichen Außendurchmesser verwendet werden. Beispielsweise kann das eigentliche Gehäuse 33 des Gasgenerators 31 mit einer Schicht 57 bestimmter Dicke umgeben sein. Je dicker die Schicht 57, um so geringer ist das sich ergebende Co-Volumen.

Wie in Fig. 4 dargestellt, kann der Gasgenerator 31 auch so ausgebildet sein, dass die abdichtende Scheibe 37 verschiebbar im Gasgenerator geführt ist. Diejenigen Gehäuseteile, die den Gasgenerator bzw. die Scheibe 37 an ihrer Rückseite beaufschlagen, um bei einer Umsetzung des Gas erzeugenden Materials 19 eine Vergrößerung des Volumens des Gasgenerators 31 zu verhindern, können verschiebbar im Gehäuse 5 geführt sein. Ein oder mehrere Anschlagringe 59 können dazu dienen, um als Anschlag für die verschiebbaren Gehäuseteile zu wirken. Je weniger Anschlagringe 59 verwendet werden, um so weiter können die verschiebbaren Gehäuseteile nach einem Einsetzen in das Gehäuse die Scheibe 37 des Gasgenerators 31 in dessen Innenraum hineindrücken und dabei das Volumen des Raums, in welchem das Gas erzeugt wird, verändern. Auf diese Weise kann ein und derselbe Gasgenerator zur Erzeugung unterschiedliche Druckverläufe verwendet werden.

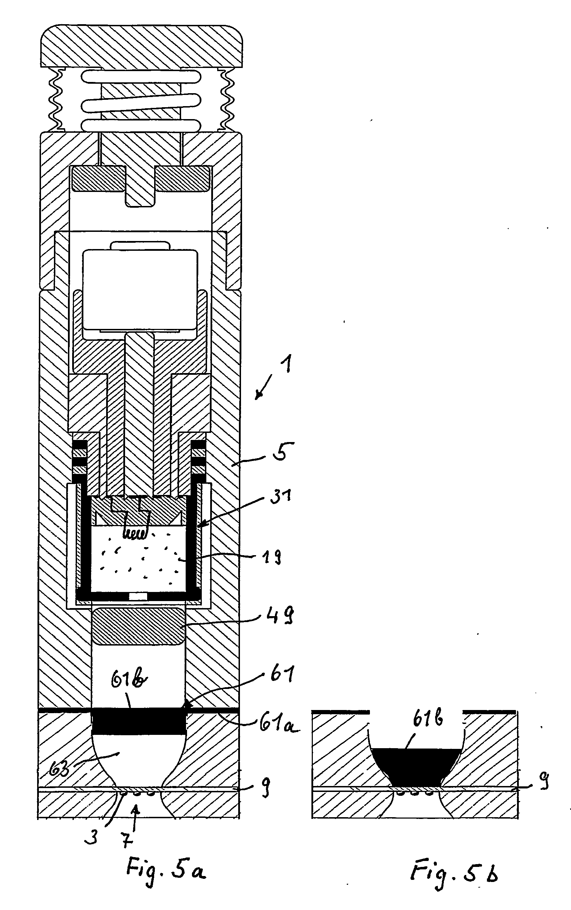

Die in Fig. 5 dargestellte Ausführungsform unterscheidet sich von der Ausführungsform nach Fig. 4 dadurch, dass zusätzlich zum Kolben 49 ein weiterer Kolben 61 verwendet wird. Dieser Kolben ist in seiner Ausgangsposition im Gehäuse fixiert und weist hierzu einen seitlichen Bereich 61 a auf, der fest im Gehäuse 5 gehalten ist. Nach dem Aktivieren des Gas erzeugenden Materials 19 wird der Kolben 49 beschleunigt und komprimiert das vor ihm und hinter dem weiteren Kolben 61 befindliche Gasvolumen. Wird ein bestimmter Druck überschritten, so bricht der eigentliche Kolbenbereich 61b des Kolbens 61 aus und wird seinerseits beschleunigt. Durch die Wahl der Druckschwelle, bei der der Kolbenbereich 61b ausbricht, kann festgelegt werden, wie schnell der ausgebrochene Kolbenbereich 61b in Richtung auf die im Gehäuse gehaltene Membran 9 beschleunigt wird. Hierdurch ergibt sich wiederum ein entsprechend steiler Druckanstieg bei der Komprimierung des Gasvolumens zwischen der Vorderseite des Kolbenbereichs 61b und der Rückseite der Membran 9. Der Bereich zwischen der Membran 9 und dem Kolbenbereich 61b ist als Auffangbereich 63 ausgebildet. Dieser dient dazu, um den ausgebrochenen und beschleunigten Kolbenbereich 61b zu verzögern und aufzufangen. Der Auffangbereich 63 ist in Richtung auf die Rückseite der Membran 9 verjüngend ausgebildet. Hierdurch wird eine Deformation des Kolbenbereichs 61b während des Auffangens erreicht. Damit ergibt sich einerseits eine abdichtende Wirkung und andererseits wird während des Auffangens gleichzeitig die Vorderseite des Kolbenbereichs 61b gegenüber dem Massenschwerpunkt beschleunigt. Hierdurch ergibt sich eine Beschleunigung des vorderen verjüngten Teils des Kolbens 61 und damit eine höhere Aufprallgeschwindigkeit des Kolbenmaterials auf die Membran 9. Hierdurch wird ein Aufsteilen des Druckanstiegs erreicht, der auf die Membran 9 übertragen wird. Die Funktionsweise des Ablösens des zu injizierenden Stoffs 3 von der Oberfläche der Membran 9 erfolgt wie vorstehend beschrieben.

Wie in Fig. 5b dargestellt, kann sich der deformierte und aufgefangene Kolbenbereich in seiner Endstellung praktisch unmittelbar an der Rückseite der Membran 9 befinden. Gegebenenfalls kann dieser auch auf die Membran 9 auftreffen und auf diese Weise einen (zusätzlichen) Impuls auf die Membran übertragen.

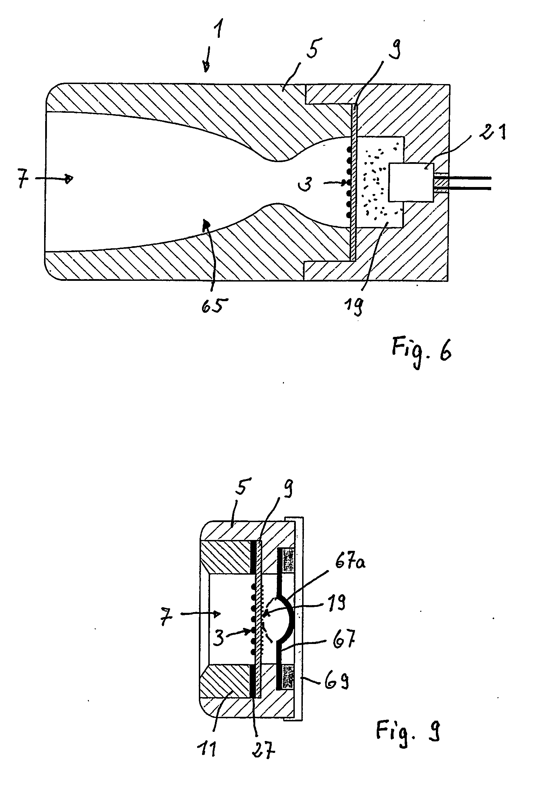

Fig. 6 zeigt eine nicht erfindungsgemäße Vorrichtung 1 zum nadellosen Injizieren eines staub- oder pulverförmigen Stoffs 3, welcher auf einer in einem Gehäuse 5 gehaltenen Membran 9 angeordnet ist. Ähnlich wie bei der Ausführungsform nach Fig. 2 wird hier ein Anzündstück 21 verwendet, um ein Gas erzeugendes Material 19 anzuzünden, welches in einem Raum vorgesehen ist, in welchem das Anzündstück 21 hineinragt und welcher mittels der Membran 9 verschlossen ist.

Im Unterschied zu allen anderen, vorstehend erläuterten erfindungsgemäßen Ausführungsformen erfolgt bei der Vorrichtung nach Fig. 6 jedoch eine Zerstörung der Membran. Hierzu ist die Membran in Bezug auf die Art und Menge des Gas erzeugenden Materials entsprechend gewählt. Die Membran kann hierzu beispielsweise aus einer dünnen Kunststofffolie bestehen, welche als Träger für den Stoff 3 dient. Auch bei einem Zerstören der Membran 9 wird der Stoff 3 von deren Oberfläche abgelöst und in Richtung auf die Austrittsöffnung 7 beschleunigt. Zur weiteren Beschleunigung der Partikel des Stoffs 3, die vom erzeugten Gas mitgerissen werden, ist der vordere Bereich des Gehäuses 5 als Düsenbereich 65 ausgebildet. Dieser kann, wie in Fig. 6 dargestellt die Form einer Laval-Düse aufweisen.

Da bei dieser Ausführungsform die Partikel des zu injizierenden Stoffs 3 vom erzeugten Gas (quasi aerodynamisch) mitgerissen und beschleunigt werden müssen, muss selbstverständlich auch eine hierzu ausreichende Gasmenge erzeugt werden.

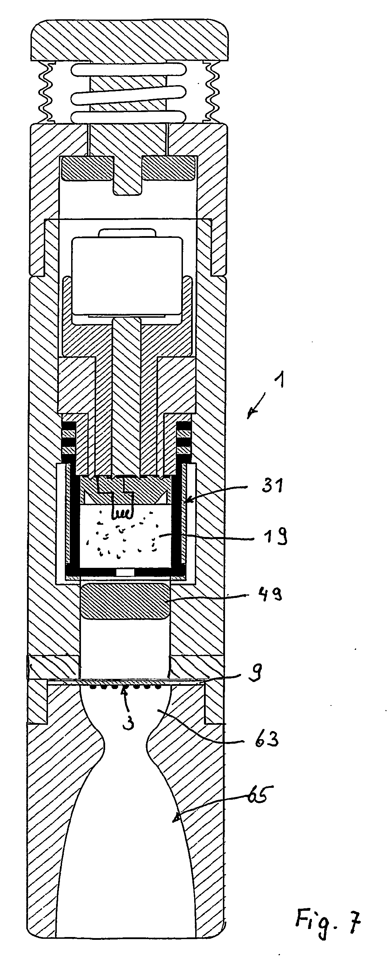

Die in Fig. 7 dargestellte Vorrichtung zum nadellosen Injizieren eines staub- oder pulverförmigen Stoffs stimmt in ihrem rückwärtigen Bereiche konstruktiv weitestgehend mit der Ausführungsform nach Fig. 4 überein. Der vordere Bereich, in welchem die Membran 9 gehalten ist, weist wieder einen Düsenbereich 65 auf, in welchen die Partikel des zu injizierenden Stoffs nach einem Zerstören der Membran 9 beschleunigt werden. Die Zerstörung der Membran 9 erfolgt durch die Beschleunigung des Kolbens 49 mittels der vom Gasgenerator 31 erzeugten Gase. Die Zerstörung der Membran erfolgt entweder bereits durch den Druck des vom Kolben 49 komprimierten Gasvolumens zwischen Kolben und Membran oder durch das Auftreffen des Kolbens 49 auf die Membran 9. Vor dem Düsenbereich 65 und in Ausstoßrichtung nach der Membran 9 ist ein Auffangbereich 63 für den Kolben 49 vorgesehen. Die Funktion dieses Auffangbereichs wurde bereits in Verbindung mit Fig. 5 erläutert. Im Unterschied zu der Ausführungsform nach Fig. 5 erfolgt die Zerstörung hier jedoch durch den vom Kolben erzeugten Gasdruck bzw. durch einen von Anfang an lose im Gehäuse geführten Kolben, der nicht ausgebrochen werden muss. Selbstverständlich wäre jedoch auch die Verwendung eines ausbrechbaren Kolbens möglich.

Durch die verjüngende Form und die hierdurch verursachte Deformation des Kolbens während des Auffangens (hierzu wird der Kolben vorzugsweise aus einem relativ weichen deformierbaren Material hergestellt), folgt wiederum eine Beschleunigung der Vorderseite des Kolbens gegenüber dessen Massenschwerpunkt und damit eine Erhöhung des Drucks bzw. ein steilerer Druckverlauf der ausgestoßenen Gase.

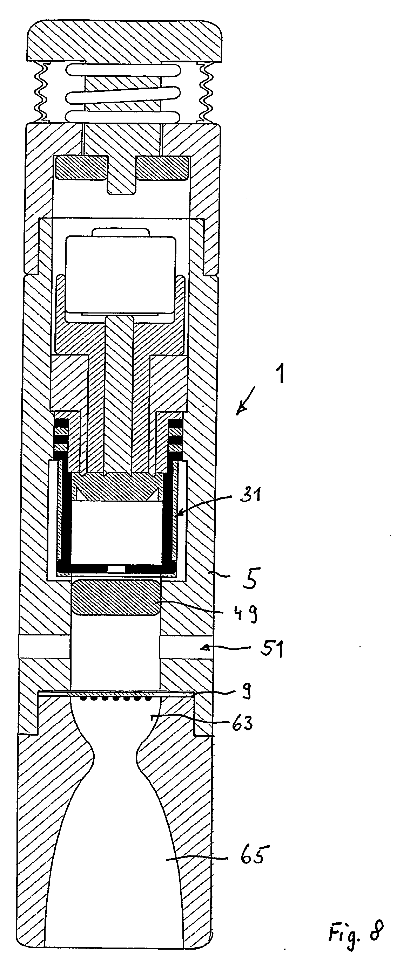

Das Beispiel nach Fig. 8 unterscheidet sich lediglich dadurch, dass im Bereich zwischen Kolben 49 und Membran 9 Ausströmöffnungen 51 vorgesehen sind. Damit erfolgt eine wesentliche Kompression des Gasvolumens zwischen Kolben und Membran erst dann, wenn der Kolben die Ausströmöffnung 51 passiert hat. Damit lässt sich ein steilerer Druckverlauf erreichen.

Die Vorrichtung in Fig. 9 entspricht hinsichtlich ihrer Wirkungsweise der Ausführungsform in Fig. 3. Die Membran 9 ist verschiebbar im Gehäuse 5 gehalten, wobei zwischen einem Haltering 11 und der Membran 9 ein kompressibles ringförmiges Element 27 vorgesehen ist. Bei dieser Vorrichtung ist jedoch das Gas erzeugende Material 19 an der der Austrittsöffnung 7 abgewandten Oberfläche der Membran 19 aufgebracht. Das Aktivieren dieses schlagempfindlichen Materials erfolgt mittels einer mechanischen Aktivierungseinrichtung, die aus einem Federplättchen 67 besteht, welches eine zentrische konvexe Wölbung 67a aufweist. Die Wölbung 67a ist im Ausgangszustand so gewölbt, wie dies in Fig. 9 dargestellt ist Die Wölbung 67a kann mittels eines nicht näher dargestellten Betätigungselements beaufschlagt werden und springt bei Überschreiten einer bestimmten Betätigungskraft in eine konvexe gespiegelte Stellung um, die in Fig. 9 gestrichelt eingezeichnet ist. Hierdurch erhält die Membran einen kurzen Schlag, wodurch das Material 19 auf der Oberfläche der Membran aktiviert wird. Die Betätigung des Federplättchens 67 kann durch eine flexible Abdeckplatte 69 hindurch erfolgen.

Durch das Aktivieren des Gas erzeugenden Materials 19 werden durch den Rückstoß impulsartige Kräfte auf die Membran ausgeübt. Diese und gegebenenfalls zugleich ein Druckanstieg im Raum zwischen der Membran und dem Federplättchen 67 führen zu einer Beschleunigung und Verschiebung der Membran 9 infolge einer Kompression des Elements 27. Durch die anschließende Verzögerung der Membran werden die Partikel des Stoffs 3 in der bereits beschriebenen Weise abgelöst.

Selbstverständlich kann die Membran 9 auch, erfindungsgemäß wie vorstehend erläutert, fest im Gehäuse gehalten sein, wobei ein Ablösen des Stoffs 3 durch eine Deformation der Membran 9 erfolgen kann.

Abschließend sei darauf hingewiesen, dass sämtliche Merkmale, die vorstehend in Verbindung mit einer bestimmten Ausführungsform erläutert sind, auch in sinnvoller Weise mit anderen Ausführungsformen kombinierbar sind.

## Patentansprüche

1. Vorrichtung zum nadellosen Injizieren eines staub- oder pulverförmigen Stoffs in ein Gewebe eines Körpers
a) mit einem Gehäuse (5), welches eine Austrittsöffnung (7) für den zu injizierenden staub- oder pulverförmigen Stoff (3) aufweist und
b) in welchem ortsfest eine Membran (9) gehalten ist, welche auf der der Austrittsöffnung (7) zugekehrten Oberfläche den staub- oder pulverförmigen Stoff (3) trägt,
c) mit einer Einrichtung zur Erzeugung eines Druckstoßes oder eines steilen Druckanstiegs zum Zweck der Ablösung des staub- oder pulverförmigen Stoffs (3) von der Membran (9), ohne dass diese zerstört wird,
**dadurch gekennzeichnet,**
d) **dass** der staub- oder pulverförmige Stoff (3) durch Adhäsion oder mittels eines Haftvermittlers auf der eben ausgebildeten Membran (9) gehalten ist,
e) **dass** die Einrichtung zur Erzeugung eines Druckstoßes ein im Gehäuse vorgesehenes aktivierbares, Gas erzeugendes Material umfasst, welches als pyrotechnisches Material oder als explosionsfähiges Gasgemisch ausgebildet ist, sowie
f) eine Aktivierungseinrichtung (17, 21, 67) für das Aktivieren des Gas erzeugenden Material (19),
g) wobei das Gas erzeugende Material (19) hinsichtlich Menge und Beschaffenheit so gewählt ist und die Membran (9) so ausgebildet und im Gehäuse (5) gehalten ist, dass bei einem Aktivieren des Gas erzeugenden Materials (19) infolge eines definierten Druckstoßes ein definierter Impuls mittelbar oder unmittelbar auf die Membran (9) übertragen wird,
h) wobei der auf die Membran (9) übertragene Impuls so bestimmt ist, das ein ausreichender Impuls auf den staub- oder pulverförmigen Stoff (3) übertragen wird, der dazu führt, dass sich Stoffpartikel des staub- oder pulverförmigen Stoffs (3) von der Membran (9) ablösen und so hoch beschleunigt werden, dass die Stoffpartikel eine ausreichende Geschwindigkeit aufweisen, um mindestens um eine vorbestimmte Tiefe in das Gewebe eindringen zu können.

2. Vorrichtung nach Anspruch 1, **dadurch gekennzeichnet, dass** das Gas erzeugende Material (19) in einer Kammer des Gehäuses (5) angeordnet ist, wobei nach dem Aktivieren des Gas erzeugenden Materials (19) ein Druckstoß oder ein steiler Druckanstieg erzeugt wird.

3. Vorrichtung nach Anspruch 2, **dadurch gekennzeichnet, dass** der Druckstoß oder der steile Druckanstieg unmittelbar auf die Membran (9) übertragen wird und bei Auftreffen auf die Membran einen ausreichenden Impuls auf diese überträgt.

4. Vorrichtung nach Anspruch 2, **dadurch gekennzeichnet, dass** mittels des Druckstoßes oder mittels des steil ansteigenden Gasdrucks ein im Gehäuse geführtes Kolbenelement (49) beaufschlagt wird, welches hierdurch so hoch beschleunigt wird, dass es bei einem Auftreffen auf die Membran (9) den erforderlichen Impuls auf diese überträgt.

5. Vorrichtung nach Anspruch 4, **dadurch gekennzeichnet, dass** das Kolbenelement (49) so ausgebildet ist, dass es auch während seiner Verschiebebewegung den Raum, dem es mit seiner durch den Gasdruck beaufschlagten Seite zugewandt ist, abdichtet, wobei das Kolbenelement (49) hierzu vorzugsweise im Bereich dieser Seite selbstlidernd ausgebildet ist.

6. Vorrichtung nach Anspruch 4 oder 5, **dadurch gekennzeichnet, dass** das Kolbenelement (49) im Gehäuse (5) so gehalten oder angeordnet ist, dass es erst bei Beaufschlagung mit einem Druck, welcher einen vorbestimmten Schwellenwert überschreitet, seine Haltekraft überwindet und schlagartig beschleunigt wird oder dass das Kolbenelement (61) mit einem Haltebereich (61 a) im Gehäuse (5) gehalten ist und bei Beaufschlagung mit einem Druck, welcher einen vorbestimmten Schwellenwert überschreitet, ein Kolbenbereich (61b) des Kolbenelements (61) ausbricht und schlagartig beschleunigt wird.

7. Vorrichtung nach einem der Ansprüche 4 bis 6, **dadurch gekennzeichnet, dass** das Kolbenelement (49, 61) infolge seiner Verschiebebewegung im Raum zwischen ihm und der Membran einen steilen Druckanstieg oder einen Druckstoß erzeugt.

8. Vorrichtung nach einem der vorhergehenden Ansprüche, **dadurch gekennzeichnet, dass** die Membran (9) aus einem Material besteht, so dimensioniert und so im Gehäuse (5) gehalten ist, dass die Schallgeschwindigkeit in der Ausstoßrichtung kleiner ist als die Geschwindigkeit des auf sie auftreffenden Druckstoßes.

9. Vorrichtung nach einem der vorhergehenden Ansprüche, **dadurch gekennzeichnet, dass** die Membran (9) zumindest in einem Teilbereich so flexibel ausgebildet ist, dass sie durch den steilen Druckanstieg, den auf sie auftreffenden Druckstoß oder das auf sie auftreffende Kolbenelement (49, 61) so deformiert wird, dass bei einer Verzögerung der betreffenden Membranbereiche auf die Partikel des zu injizierenden Stoffs (3) negative Beschleunigungskräfte wirken, dass eine Ablösung der Partikel von der Membran (9) und ein Ausstoßen der Partikel aus dem Gehäuse erfolgt.

10. Vorrichtung nach Anspruch 9, **dadurch gekennzeichnet, dass** die Membran (9) im Bereich, in dem der zu injizierende staub- oder pulverförmige Stoff aufgebracht ist, so dick ausgebildet ist, dass im Wesentlichen keine Deformation dieses Bereichs bei einer Übertragung des erzeugten Impulses auf die Membran (9) erfolgt.

11. Vorrichtung nach einem der vorhergehenden Ansprüche, **dadurch gekennzeichnet, dass** in Ausstoßrichtung des zu injizierenden Stoffs (3) vor der Membran (9) ein in Richtung senkrecht zur Membranebene kompressibles Element (27) angeordnet ist, welches als bei Übertragung eines Impulses auf die verschiebbar im Gehäuse geführte Membran (9) von dieser komprimiert wird, wobei das kompressiblc Element (27) vorzugsweise aus einem weichen Kunststoff besteht und gleichzeitig als Dichtclement dient.

12. Vorrichtung nach einem der vorhergehenden Ansprüche, **dadurch gekennzeichnet, dass** im Gehäuse (5) ein vorzugsweise separat ausgebildeter Detonator oder ein vorzugsweise separat ausgebildetes Anzündstück angeordnet ist, in welchem das pyrotechnische Material vorgesehen ist.

13. Vorrichtung nach einem der vorhergehenden Ansprüche, **dadurch gekennzeichnet, dass** das Gas erzeugende Material (19) in einem separat ausgebildeten und austauschbar im Gehäuse gehaltenen Gasgenerator (31) angeordnet ist.

14. Vorrichtung nach einem der vorhergehenden Ansprüche, **dadurch gekennzeichnet, dass** das Gas erzeugende Material (19) auf der der Ausstoßöffnung abgewandten Oberfläche der Membran (9) angeordnet ist und vorzugsweise so gewählt ist, dass es mittels eines kurzen mechanischen oder thermischen Impulses aktivierbar ist.

15. Vorrichtung nach Anspruch 14, **dadurch gekennzeichnet, dass** der thermische Impuls von einer starken Lichtquelle erzeugt wird, beispielsweise einem Laser.

## Claims

1. Device for injecting a dust-like or powder-like substance into a body tissue without using a needle
a) with a housing (5) which comprises a discharge opening (7) for the dust-like or powder-like substance (3) to be injected and
b) in which a membrane (9) is fixedly held which supports the dust-like or powder-like substance (3) on the surface facing the discharge opening (7),
c) with a device for generating a burst of pressure or a sharp increase in pressure so as to detach the dust-like or powder-like substance (3) from the membrane (9) without damaging said membrane,
**characterised in that**
d) the dust-like or powder-like substance (3) is held on the planar membrane (9) by adhesion or by means of an adhesive agent,
e) the device for generating a burst of pressure comprises an activatable, gas-generating material provided in the housing, which material is a pyrotechnic material or an explosive gas mixture, as well as
f) an activation device (17, 21, 67) for activating the gas-generating material (19),
g) the quantity and condition of the gas-generating material (19) being selected and the membrane (9) being configured and held in the housing (5) in such a way that, when the gas-generating material (19) is activated as a result of a defined burst of pressure, a defined pulse is transmitted indirectly or directly to the membrane (9),
h) the pulse transmitted to the membrane (9) being determined in such a way that a sufficient pulse is transmitted to the dust-like or powder-like substance (3) which leads to substance particles of the dust-like or powder-like substance (3) detaching from the membrane (9) and being accelerated to such an extent that the substance particles are fast enough to be able to penetrate the tissue at least as far as a predetermined depth.

2. Device according to claim 1, **characterised in that** the gas-generating material (19) is arranged in a chamber of the housing (5), a burst of pressure or a sharp increase in pressure being generated after the gas-generating material (19) has been activated.

3. Device according to claim 2, **characterised in that** the burst of pressure or the sharp increase in pressure is directly transmitted to the membrane (9) and; when impacting the membrane, transmits a sufficient pulse thereto.

4. Device according to claim 2, **characterised in that** a piston element (49) guided in the housing is loaded by means of the burst of pressure or by means of the sharply increasing gas pressure, which piston element is thus accelerated to such a degree that when it impacts on the membrane (9) it transmits the necessary pulse thereto.

5. Device according to claim 4, **characterised in that** the piston element (49) is configured in such a way that it seals the space which it faces with its side impacted by the gas pressure even during its displacement, the piston element (49) being configured in a self-obturating manner for this, preferably in the region of this side.

6. Device according to either claim 4 or claim 5, **characterised in that** the piston element (49) is held or arranged in the housing (5) in such a way that, when it is impacted by a pressure which exceeds a predetermined limit, it overcomes its retaining force and is abruptly accelerated, or **in that** the piston element (61) is held in the housing (5) by a retaining region (61a) and, when impacted by a pressure which exceeds a predetermined limit, a piston region (61b) of the piston element (61) breaks away and is abruptly accelerated.

7. Device according to any one of claims 4 to 6, **characterised in that** the piston element (49, 61) generates a sharp increase in pressure or a burst of pressure as a result of its displacement in the space between itself and the membrane.

8. Device according to any one of the preceding claims, **characterised in that** the membrane (9) is made of such a material, is so dimensioned and is held in the housing (5) in such a way that the acoustic velocity in the discharge direction is less than the velocity of the burst of pressure impacting said material.

9. Device according to any one of the preceding claims, **characterised in that** the membrane (9) is configured so as to be flexible, at least in a particular region, in such a way that it is deformed by the sharp increase in pressure, the burst of pressure impacting it or the piston element (49, 61) impacting it in such a way that, during a deceleration of the membrane regions concerned, negative acceleration forces act on the particles of the substance (3) to be injected in such a way that the particles are separated from the membrane (9) and discharged from the housing.

10. Device according to claim 9, **characterised in that** the membrane (9), in the region in which the dust-like or powder-like substance to be injected is applied, is so thick that said region remains substantially undeformed when the generated pulse is transmitted to the membrane (9).

11. Device according to any one of the preceding claims, **characterised in that** ahead of the membrane (9) in the discharge direction of the substance (3) to be injected, an element (27) is arranged which can be compressed in a direction perpendicular to the membrane plane and which, upon transmission of an impulse to the membrane (9), which is guided displaceably in the housing, is compressed by said membrane, the compressible element (27) preferably being made of a flexible plastics material and also being used as a sealing element.

12. Device according to any one of the preceding claims, **characterised in that** in the housing (5) a preferably separate detonator or a preferably separate igniting portion is arranged, in which the pyrotechnic material is provided.

13. Device according to any one of the preceding claims, **characterised in that** the gas-generating material (19) is arranged in a separate gas generator (31) which can be replaced and is held in the housing.

14. Device according to any one of the preceding claims, **characterised in that** the gas-generating material (19) is arranged on the surface of the membrane (9) remote from the discharge opening and is preferably selected in such a way that it can be activated by means of a short mechanical or thermal pulse.

15. Device according to claim 14, **characterised in that** the thermal pulse is generated by a strong light source, for example a laser.

## Revendications

1. Dispositif pour injecter sans aiguille une substance pulvérulente ou poudreuse dans un tissu d'un corps,
a) avec un boîtier (5), présentant une ouverture de sortie (7) pour la substance pulvérulente ou poudreuse (3) à injecter, et
b) boîtier dans lequel est maintenue de façon localement fixe une membrane (9) portant, sur la surface tournée vers l'ouverture de sortie (7), la substance pulvérulente ou poudreuse (3),
c) avec un dispositif de génération d'un choc de pression ou une augmentation à croissance à gradient élevé de la pression, dans le but de détacher la substance pulvérulente ou poudreuse (3) de la membrane (9), sans que celle-ci soit détruite,
**caractérisé en ce que**
d) la substance pulvérulente ou poudreuse (3) est maintenue sur la membrane (9) plane par adhésion ou au moyen d'un adhésif,
e) le dispositif de génération d'un choc de pression comprend un matériau générant un gaz, activable, prévu dans le boîtier, réalisé sous forme de matériau pyrotechnique ou de mélange de gaz explosible, et
f) un dispositif d'activation (17, 21, 67) pour provoquer l'activation du matériau (19) générant un gaz,
g) où le matériau (19) générant un gaz est choisi, quant à la quantité et à la qualité, et la membrane (9) est réalisée et maintenue dans le boîtier (5), de manière que, lors d'une activation du matériau (19) générant un gaz, par suite d'un choc de pression défini, une impulsion définie soit transmise, indirectement ou directement, à la membrane (9),
h) où l'impulsion transmise à la membrane (9) est déterminée de manière qu'une impulsion suffisante soit transmise à la substance pulvérulente ou poudreuse (3), menant à ce que des particules de substance de la substance pulvérulente ou poudreuse (3) se détachent de la membrane (9) et soient accélérées si fortement que les particules de substance présentent une vitesse suffisante pour pouvoir pénétrer au moins de la valeur d'une profondeur prédéterminée dans le tissu.

2. Dispositif selon la revendication 1, **caractérisé en ce que** le matériau (19) produisant du gaz est disposé dans une chambre du boîtier (5), un choc de pression ou une augmentation à gradient élevé de pression étant produit(e) après activation du matériau (19) produisant du gaz.

3. Dispositif selon l'une des revendications précédentes, **caractérisé en ce que** le choc de pression ou l'augmentation à gradient élevé de pression est transmis(e) directement à la membrane (9) et, lors de l'arrivée sur la membrane, transmet une impulsion suffisante à celle-ci.

4. Dispositif selon la revendication 2, **caractérisé en ce qu'**au moyen du choc de pression ou au moyen de l'augmentation à gradient élevé de pression de gaz, un élément formant piston (19), guidé dans le boîtier, est sollicité, étant de ce fait si fortement accéléré que, lors de son arrivée sur la membrane (9), il transmet l'impulsion nécessaire à celle-ci.

5. Dispositif selon la revendication 4, **caractérisé en ce** l'élément formant piston (49) est réalisé de manière que, également pendant son mouvement de déplacement, il isole hermétiquement l'espace vers lequel il est tourné par sa face sollicitée par la pression de gaz, l'élément formant piston (49) étant à cette fin de préférence réalisé de façon auto-glissante dans cette zone.

6. Dispositif selon la revendication 4 ou 5, **caractérisé en ce** l'élément formant piston (49) est maintenu ou disposé dans le boîtier (5), de manière que, ensuite, lors de la sollicitation par une pression dépassant une valeur de seuil prédéterminée, il surmonte sa force de maintien et est accéléré d'un coup ou que l'élément formant piston (61) est maintenu dans le boîtier (5) par une zone de maintien (61a) et, lors de la sollicitation par une pression dépassant une valeur de seuil prédéterminée, une zone de piston (61 b) de l'élément formant piston (61) se rompt et est accélérée d'un coup.

7. Dispositif selon l'une des revendications 4 à 6, **caractérisé en ce** l'élément formant piston (49, 61), par suite de son déplacement d'accélération, produit dans l'espace situé entre lui et la membrane une augmentation de pression à gradient élevé ou un choc de pression.

8. Dispositif selon l'une des revendications précédentes, **caractérisé en ce** la membrane (9) est formée d'un matériau, dimensionné et maintenue dans le boîtier (5), de manière que la vitesse du son dans la direction d'expulsion soit inférieure à la vitesse du choc de pression arrivant sur lui.

9. Dispositif selon l'une des revendications précédentes, **caractérisé en ce**, dans une zone partielle, la membrane est réalisée avec une flexibilité telle que, de par l'augmentation de pression à gradient élevé, le choc de pression arrivant sur elle, ou l'élément formant piston (49, 61) arrivant sur elle, elle est déformée à un degré tel que, dans le cas d'un ralentissement des zones de membrane concernées, sur les particules de la substance (3) à injecter agissent des efforts d'accélération négatifs, faisant qu'un détachement des particules vis à vis de la membrane (9) et une expulsion des particules hors du boîtier se produisent.

10. Dispositif selon la revendication 9, **caractérisé en ce que**, dans la zone dans laquelle la substance pulvérulente ou poudreuse est appliquée, la membrane (9) est d'une épaisseur telle que pratiquement aucune déformation de cette zone ne se produit lors d'une transmission de l'impulsion produite à la membrane (9).

11. Dispositif selon l'une des revendications précédentes, **caractérisé en ce qu'**un élément (27) compressible perpendiculairement au plan de la membrane, élément qui, lors de la transmission d'une impulsion à la membrane (9) guidée de façon déplaçable dans le boîtier, est comprimé par celle-ci, est disposé devant la membrane (9), en observant dans la direction d'expulsion de la substance (3) à injecter, l'élément (27) compressible étant de préférence composé d'une matière synthétique souple et servant simultanément d'élément d'étanchéité.

12. Dispositif selon l'une des revendications précédentes, **caractérisé en ce qu'**un détonateur, de préférence réalisé séparément, ou une pièce d'allumage, de préférence réalisée séparément, dans lequel, ou laquelle, est prévu le matériau pyrotechnique, est disposé dans le boîtier (5).

13. Dispositif selon l'une des revendications précédentes, **caractérisé en ce que** le matériau (19) générant du gaz est disposé dans un générateur de gaz (31), réalisé séparément et maintenu de façon remplaçable dans le boîtier.

14. Dispositif selon l'une des revendications précédentes, **caractérisé en ce que** le matériau (19) générant du gaz est disposé sur la surface de la membrane (9) opposée à l'ouverture d'expulsion et est, de préférence, choisi tel qu'il soit activable au moyen d'une courte impulsion, mécanique ou thermique.

15. Dispositif selon la revendication 14, **caractérisé en ce que** l'impulsion thermique est produite par une forte source de lumière, par exemple par un laser.
